# EUROPEAN PATENT SPECIFICATION

(11) **EP 1 551 483 B1**
(45) Date of publication and mention of the grant of the patent: **10.01.2007**
(21) Application number: 03798681.7
(22) Date of filing: 30.07.2003
(51) Int. Cl.: A61M 5/50

(54) **SINGLE USE SYRINGE FOR TWO STROKE PROCEDURES**
EINWEG-SPRITZE FÜR ZWEISTUFIGE VERFAHREN
SERINGUE A USAGE UNIQUE POUR OPERATIONS EN DEUX TEMPS

(30) Priority: 25.09.2002 US 254924; 24.10.2002 US 421024 P
(43) Date of publication of application: 13.07.2005
(73) Proprietor: Becton Dickinson and Company, Franklin Lakes, New Jersey 07417-1880 (US)
(72) Inventor: PELKEY, Brian, J., Rockaway, NJ 07866 (US); FLEISCHER, Gene, New City, NY 10956 (US); SAMUEL, P.R., Suresh, Coimbatore 641 042 (IN). (IN)
(74) Representative: Weber, Thomas
(86) International application number: PCT/US2003/023839
(87) International publication number: WO 2004/028603

(56) References cited:
- EP-A- 0 308 040
- US-A- 4 961 728
- US-A- 5 989 219

## Description

### BACKGROUND OF THE INVENTION

The present invention is related to single use syringes and more particularly to auto-disable single use syringes for use in two stroke procedures.

In the United States and throughout the world, the multiple use of hypodermic syringe products that are intended for single use only is instrumental in drug abuse and more particularly in the transfer of diseases. Intravenous drug users who routinely share and reuse syringes are a high risk group with respect to the bloodbome pathogens including HIV and AIDS. Also the effects of multiple use are a major concern in under-developed countries where repeated use of syringe products may be responsible for the spread of many diseases. Reuse of single use hypodermic syringe assemblies is instrumental in the spread of drug abuse even in the absence of infection or disease.

Many attempts have been made to remedy this problem, Some of these attempts have required a specific act to destroy the syringe after use either by using a destructive device or providing a syringe assembly with frangible zones so that the syringe could be rendered inoperable by application of force. Other attempts have involved the inclusion of structure which would allow the destruction or defeating of the syringe function through a conscious act by the syringe user. Although many of these devices work quite well, they do require the specific intent of the user followed by the actual act to destroy or render the syringe inoperable.

Other prior art syringes are auto-disable syringes. Such syringes automatically disable after a single injection stroke is made. Thus, these syringes cannot be used for procedures that require more than a single aspiration stroke and a single injection stroke. For example, such syringes cannot be used for reconstitution or other applications which require more than a single stroke of the plunger rod.

### SUMMARY OF THE INVENTION

The invention relates to a syringe assembly that automatically disables after two strokes of the plunger rod. The syringe assembly includes a syringe barrel, a plunger rod having an elongate body portion, a stopper, a disc and at least one axially extending recess formed by a pair of surfaces radially displaced from the longitudinal axis of said elongate body portion. The surfaces of the plunger rod define at least one proximal tooth and at least one distal tooth. A locking element is slidably positioned within the recess and engages the inside surface of said syringe barrel such that said locking element is substantially immovable in the proximal. The locking element is further engageable with the proximal tooth during said first injection stroke of said plunger rod and engageable with the distal tooth during said second injection stroke of said plunger rod. The locking element engages the teeth of the plunger rod in such a way as to render the syringe assembly inoperable after completing two strokes of the plunger rod. An annular ring may be included on the inside barrel of the syringe to limit motion of the plunger rod in the proximal direction.

According to another aspect of the invention, the surfaces may define a plurality of proximal teeth and a plurality of distal teeth.

Preferably the locking element is an integral structure comprised of a resilient metallic material, but can be formed of any material that is resilient.

Optionally, another syringe assembly which automatically disables after two strokes of the plunger rod may be provided. Such a syringe assembly includes a barrel, a plunger rod extending including an elongate body portion, a stopper at a distal end of the elongate body portion, a disc, a first axially extending recess formed by a pair of first surfaces radially displaced from the longitudinal axis of the elongate body portion and a second axially extending recess formed by a pair of second surfaces radially displaced from the longitudinal axis of the elongate body portion. The first pair of surfaces each define a plurality of teeth and the second pair of surface each define at least one proximal tooth and at least one distal tooth. The syringe assembly further includes a locking element slidably positioned within either of the first or second recesses. Thus, a syringe assembly is provided that may be used for procedures requiring only one stroke of the plunger rod or two strokes of the plunger rod. For procedures requiring only one stroke of the plunger rod, the locking element is placed within the first recess containing a plurality of teeth. After completion of the single injection stroke, the syringe is rendered disabled by the locking element. For procedures requiring two injection strokes, the locking element is placed within the second recess and the syringe is rendered disables after two injections strokes are completed.

### BRIEF DESCRIPTION OF THE DRAWINGS

FIG. 1 is an exploded, perspective view of a single use syringe assembly according to the invention.

FIG. 2 is a perspective view of the single use syringe assembly of FIG. 1 as packaged.

FIG. 3 is a perspective view of the syringe assembly with the plunger rod in position following the first aspiration.

FIG. 4 is a perspective view of the syringe assembly with the plunger rod in position following the first injection.

FIG. 5 is a perspective view of the syringe assembly with the plunger rod in position following the second aspiration.

FIG. 6 is a perspective view of the syringe assembly with the plunger rod in position following the second injection.

FIG. 7 is a cross-sectional view of the syringe assembly as shown in FIG.FIG. 2

FIG. 7A is an exploded cross-sectional view of the syringe assembly shown in FIG. 7.

FIG. 8 is a cross-sectional view of the syringe assembly as shown in FIG. 3

FIG. 8A is an exploded cross-sectional view of the syringe assembly as shown in FIG. 8.

FIG. 9 is a cross-sectional view of the syringe assembly as shown in FIG. 4.

FIG. 9A is an exploded cross-sectional view of the syringe assembly as shown in FIG. 9.

FIG. 10 is a cross-sectional view of the syringe assembly as shown in FIG. 5.

FIG. 10A is an exploded cross-sectional view of the syringe assembly as shown in FIG. 10.

FIG. 11 is a cross-sectional view of the syringe assembly as shown in FIG. 6.

FIG. 11A is an exploded cross-sectional view of the syringe assembly as shown in FIG. 11.

FIG. 12 is a is a cross section view of the syringe assembly taken along line 10-10 of FIG. 7.

FIG. 13 is a perspective vie of the plunger rod.

FIG. 14 is a top perspective view of the locking element.

FIG. 15 is a bottom perspective view of the locking element.

FIG. 16 is a side elevation view of the locking element.

FIG. 17 is a cross-sectional view of the locking element taken along line 16-16 of FIG. 16.

FIG. 18 is a cross-sectional view of the locking element taken along line 17-17 of FIG. 16.

FIG. 19 is an end view of the locking element.

FIG. 20 is an exploded, perspective view of a single use syringe assembly according to the another embodiment of the invention.

FIG. 21 is a perspective view of the syringe assembly of FIG. 20 as packaged.

FIG. 22 is a perspective view of the syringe assembly of FIG. 20 with the plunger rod in position following the first aspiration.

FIG. 23 is a perspective view of the syringe assembly of FIG. 20 with the plunger rod in position following the first injection.

FIG. 24 is a perspective view of the syringe assembly of FIG. 20 with the plunger rod in position following the second aspiration.
FIG. 25 is a perspective view of the syringe assembly with the plunger rod in position following the second injection.

### DETAILED DESCRIPTION

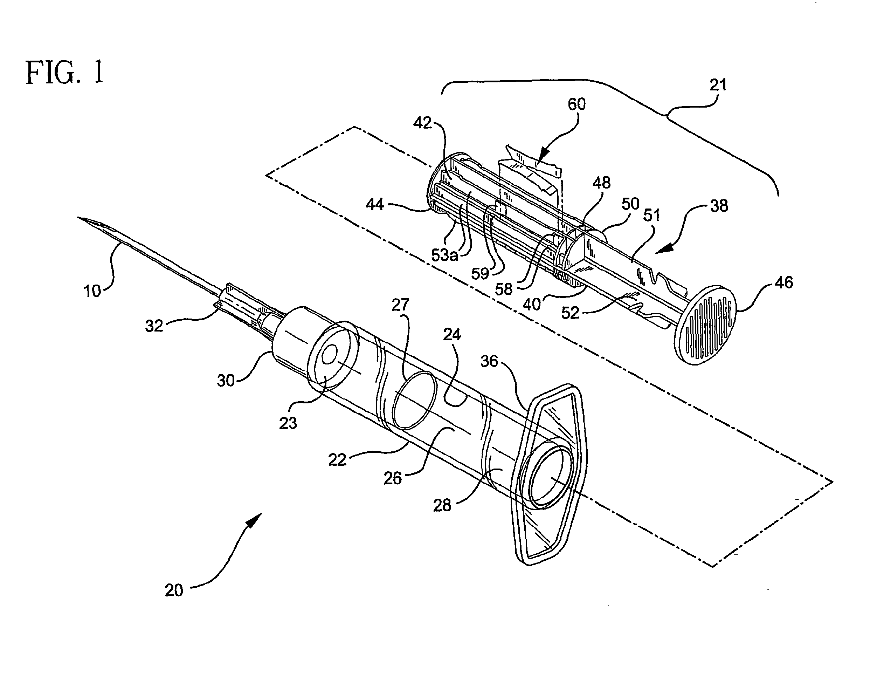

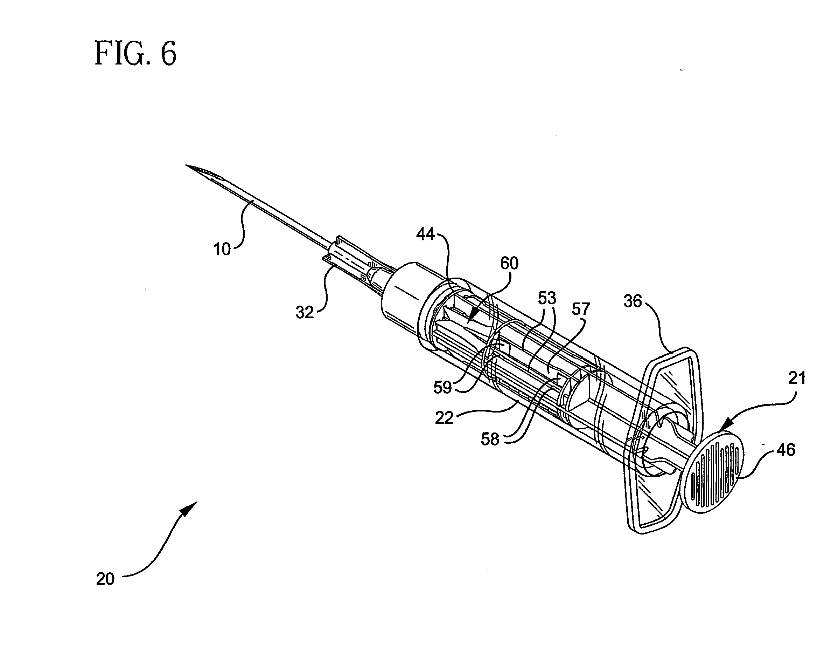

Referring to FIGS. 1-11A, a single use syringe assembly 20 includes a barrel 22 having an inside surface 24 defining a chamber 26 for retaining fluid. The inside surface 24 of the barrel further defines an annular ring 27 projecting therefrom. The barrel 22 includes an open proximal end 28 and a distal end 30 having a collar 32 defining a passageway 34 therethrough in communication with the chamber. A needle cannula 10 projects outwardly from the distal barrel end and is in fluid communication with the chamber 26. The collar 32 of the barrel as shown is a non-standard collar and therefore cannot be used with a standard needle cannula, e.g., a standard luer locking cannula. Therefore, reuse of the needle or the syringe is difficult. It will be appreciated that the invention could be applied to syringe assemblies having permanently affixed needles or needle/hub assemblies, or fixed or removable blunt cannulas, or standard luer locking assemblies. While intended for reconstitution of vaccines, the syringe assembly can be used for general curative purposes or other purposes.

As used in the preceding paragraph and hereafter, the term "distal end" refers to the end furthest from the person holding the syringe assembly 20. The term "proximal end" refers to the end closest to the holder of the syringe assembly 20. In the preferred embodiment, the proximal end 28 of the barrel 22 includes a flange 36 to facilitate handling and positioning of the syringe assembly 20 and to maintain the relative position of the barrel 22 with respect to a plunger rod 38 during filling and medication administration.

The plunger rod 38 used in the syringe assembly 20 includes an elongate body portion 40 including a plurality of elongate recesses 42 running substantially parallel to the longitudinal axis of rotation thereof. The distal end of the elongate body portion 40 includes an integral stopper 44. A disc-shaped flange 46 is provided at the proximal end of the plunger rod 38 for allowing the user to apply the force necessary to move the plunger rod 38 with respect to the barrel 22.

The elongate body portion 40 includes a distal disc 48 and a proximal disc 50 intermediate to the proximal and distal ends thereof. The sections between the relatively proximal disc 50 and the flange 46 and the relatively distal disc 48 and the stopper 44 include radially extending walls 51, 52, both of which preferably traverse the longitudinal axis of rotation of the plunger rod 38. Additional pairs of distal walls 53 resembling fins extend perpendicularly from both sides of one wall 52 of the radially extending walls in the section between the distal disc 48 and stopper 44. In this embodiment, as shown in the FIGS. 1-9, there are four pairs of distal walls 53 including wall faces 55, 57. A first pair 53a of distal walls extend perpendicularly from the upper side of wall 52 and a second pair 53b extend from the lower side of wall 52. The third and fourth pairs are mirror images of the first and second extending from the wall 52 on the side opposite from the first and second pairs beyond wall 51.

In a preferred embodiment, each of the distal walls 53 is substantially parallel to each other. The areas between each of the pairs of walls could be filled in to provide additional rigidity if necessary. Teeth 54 are formed on selected surfaces of the walls 53. In a preferred embodiment, each of the first pair 53a of distal walls defines a pair of teeth including a proximal tooth 58 and a distal tooth 59 on wall faces 55, 57. Each of the second pair 53b of distal walls defines a plurality of teeth 54b. The proximal tooth is located adjacent to the distal disc 48 while the distal tooth 59 is located at some point between the distal disc 48 and the stopper 44. An area 62 having no teeth is defined between the distal tooth 58 and proximal tooth 58. Each tooth 54, 58, 59 includes a corresponding distally facing surface or shoulder 56. The wall surfaces 55, 57 including the teeth, converge along an imaginary line that runs substantially parallel to the longitudinal axis of the plunger rod assembly, but radially displaced therefrom. As shown in the drawings, the surfaces 55, 57 do not necessarily adjoin each other. The wall surfaces 55, 57 define recesses 42 for positioning of a locking element 60. While four recesses are provided, a greater or lesser number may be employed. It will be appreciated that the recesses 42 can be formed by surfaces that actually meet along the line of convergence. It will further be appreciated that while the plunger rod assembly as shown and described herein is of integral construction, it may in fact be comprised of two or more separate elements. The stopper 44 may, for example, be a separate component made from a material that is different from the material comprising the remainder of the plunger rod assembly. Preferably, the syringe barrel is comprised of polypropylene, and contains an internal lubricant and the plunger rod assembly is comprised of polyethylene.

According to one embodiment of the invention, the diameter of the barrel is that of a standard 10 ml 2 piece syringe, at least about 13.5 mm (0.53 inch). For delivery of smaller doses, for example, 2ml, 3ml, 5ml, the length of the syringe is reduced from the length of the standard 10 ml syringe to the length required to deliver the smaller dose. Thus, the bulk of the syringe is reduced, while the pressure can be more easily controlled due to the greater diameter of the barrel. For delivery of larger doses, for example, 10ml, the diameter of a standard 20ml barrel, or larger barrel, can be used, while, again reducing the length of the syringe to accommodate the smaller dose. The reduction in the bulk of the syringe reduces the packaging, which is advantageous in syringes having an integral cannula. The diameter of the plunger is also increased, increasing the pressure it exerts. This improves the reconstitution process.

The locking element 60 is positioned within the barrel 22 and within one of the elongate recesses 42 defined by the pairs of distal walls 53. As shown in the drawings, the locking element 60 is placed within the first pair of walls 53a which each define distal and proximal teeth 58, 59. When the locking element 60 is placed within either of the first pair of distal walls 53a, the syringe may be used for reconstitution procedures, or any other procedure requiring two strokes of the plunger since there are two teeth 58, 59. This operation is set out in more detail below. Alternatively, the locking element 60 may be placed within the second pair of distal walls 53b, which define a plurality of teeth 54. When placed in this location, the plunger is only capable of a single injection stroke, and therefore, the syringe 20 cannot be used for procedures requiring more than one aspiration and injection stroke. This operation is set out in more detail below. The recess 42 acts as a pathway for longitudinal motion of the locking element 60 relative to the plunger rod assembly 38. Since the recesses 42 are displaced from the longitudinal axis of the plunger rod assembly 38, the same size locking element 60 can be used that is employed in a smaller syringe. In the smaller syringe, the locking element would be positioned in a recess adjoining the longitudinal axis or at least closer to this axis than in the syringe disclosed herein. U.S. Patent Nos. 4,961,728 and 5,989,219 disclose the placement of a locking element at or near the longitudinal axis. A syringe as disclosed in these patents could be provided for administering doses of about 0.5ml. The invention allows the same size locking element to be used in a wide range of syringes, such as very small syringes (0.5ml) as well as those exceeding five milliliters (5ml).

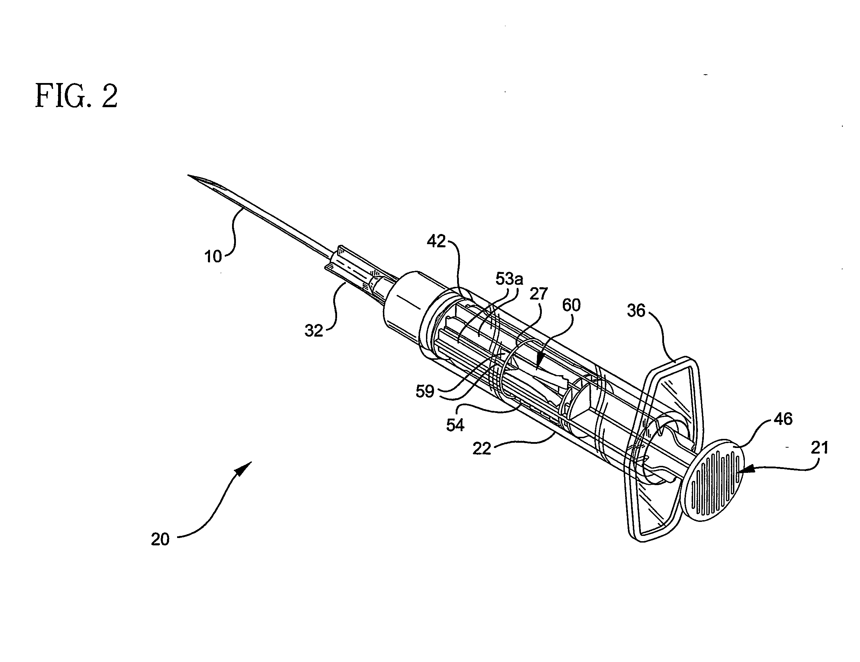

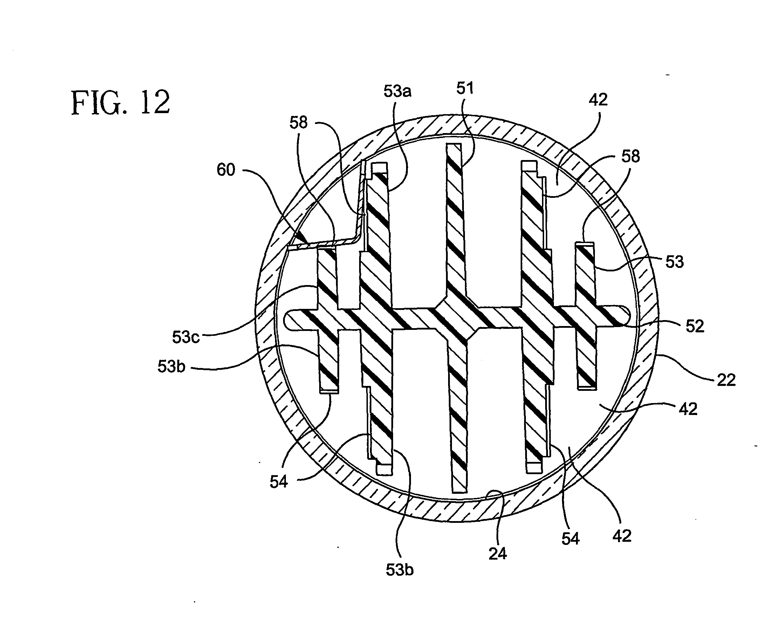

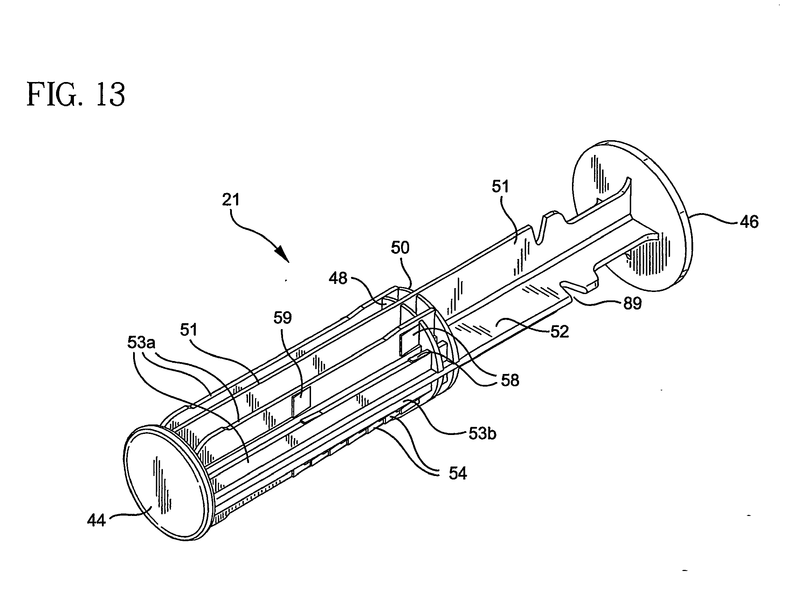

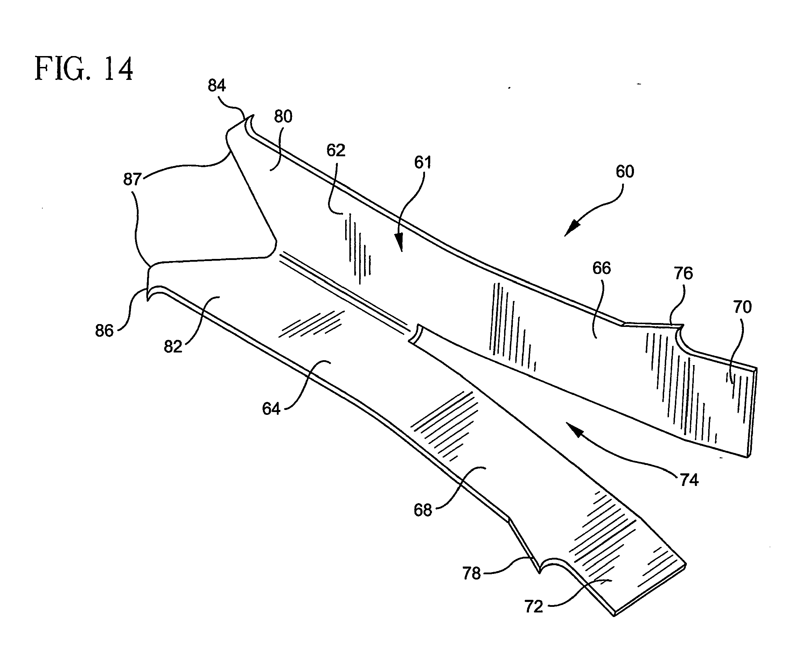

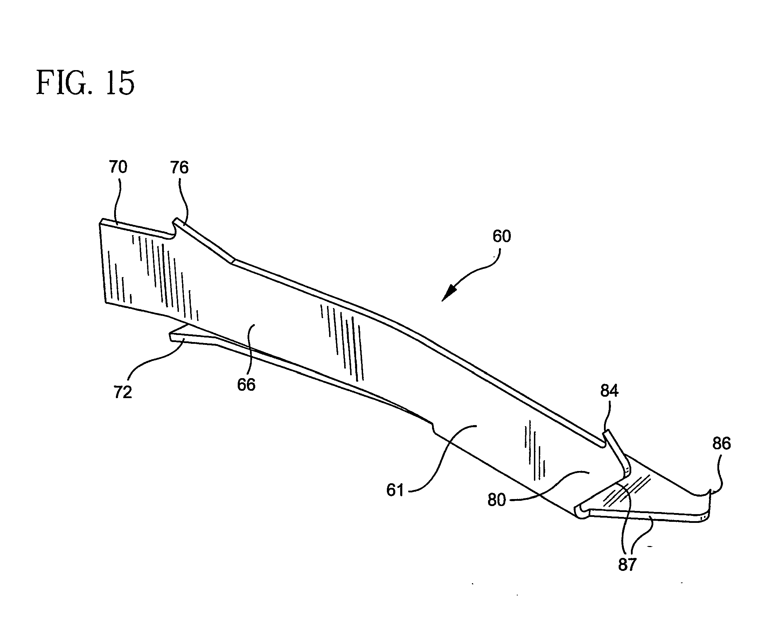

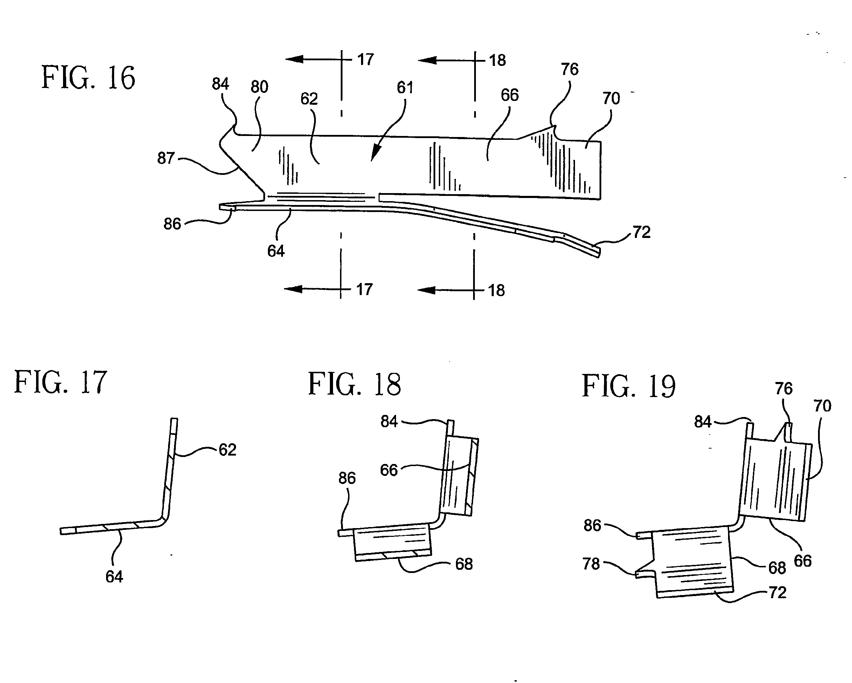

The locking element 60, as best shown in FIGS. 12-18, includes a generally V-shaped body portion 61 comprising first and second radially extending walls 62, 64 joined along a longitudinal axis. The walls 62, 64 preferably form an angle of greater than ninety degrees, and preferably about one hundred degrees. A first leg 66 extends proximally from the first wall 62 and a second leg 68 extends proximally from the second wall 64. The legs 66,68 flare outwardly with respect to the V-shaped body portion 61, as best shown in FIG. 2. The legs 66, 68 are preferably substantially longer than the length of the body portion 61. For example, in a locking element having an overall length of about seventeen millimeters, the legs 66, 68 may be about ten millimeters in length.

Each of the legs 66, 68 includes a proximal end portion 70, 72 that is angled towards one of the walls 53 of the plunger rod. They further include inner and outer edges. (The terms "inner" and "outer" are relative terms as used herein.) The inner edges thereof are substantially adjacent to each other, separated by a longitudinal gap 74. Barbs 76, 78 are integral with the outer edges of the first and second legs. The barbs face proximally, and are preferably located slightly distally of the angled end portions 70, 72. The barbs may be different in appearance from those shown in the drawings so long as they are capable of engaging the inside surface 24 of the syringe barrel to prevent proximal movement of the locking element 60.

A second pair of legs 80, 82 extends distally from the V-shaped body portion 61. One of these legs 80 extends from the first wall 62 and the other 82 from the second wall 64. Barbs 84, 86 extend proximally from the distal ends of the legs 80, 82. The barbs 84,86 are formed on the outer edges of the distally extending legs 80,82. Each leg further includes a cutting edge 87 capable of penetrating the stopper 44.

The locking element 60 is preferably formed from a thin sheet of metal such as stainless steel. The thickness in a preferred embodiment thereof is about 0.20 mm. The dimensions of the locking element 60 are selected in accordance with the barrels and plunger rod assemblies with which it is to be used. The angle formed between the two halves of the locking element 60, as shown in FIG. 12, is preferably greater than ninety degrees, and preferably about one hundred degrees. When placed in one of the recesses 42 in the plunger rod, the locking element will accordingly exert a force against the two of the converging wall surfaces 55, 57 that define the recess. The cutting edges 87 are preferably formed by providing bevels on one side of the substrate from which the locking element is constructed. It will be appreciated that the substrate could be ground on both sides thereof to form cutting edges 87 for disabling the stopper 44. Other methods for creating cutting edges could also be employed, such as photochemical machining, which are well known in the art. Alternatively, barbs (not shown) or other cutting members can be provided on the locking element for piercing the stopper.

The syringe assembly is easily constructed from the component parts thereof and packaged as shown in FIGS. 1 and 2. First, the locking element 60 is positioned in one of recesses 42 in the plunger rod such that the angled end portions of the legs 66, 68 adjoin the relatively distal disk 48. As shown in FIGS. 1 and 2, the locking element 60 is positioned in the recess 42 formed by the first pair of distal walls 53a. In this position, the syringe may be used for a reconstitution process as will be illustrated. The legs 66, 68 and spring member extend proximally, and the barbs 76, 78, 84, 86 are all angled proximally with respect to the plunger rod 38. The plunger rod/locking element assembly 21 is then inserted into the barrel 22 through the proximal end thereof.

As the assembly 21 is moved distally within the barrel, the angular orientation of the barbs allows them to slide along while engaging inside surface 24 of the barrel. The locking element 60 moves distally with the plunger rod 38 due to the engagement of the ends of legs 66, 68 with the disc 48. The gap 74 is maintained between the legs 66, 68 even after installation of the locking element. The maintenance of the gap and the relatively long lengths of the legs 66,68, which act as cantilever springs, provide a relatively reduced force on the barrel and facilitate use and installation. The plunger rod/locking element assembly 21 is moved distally until the stopper 44 engages an end wall 23 of the barrel 22. The annular ring 27 is spaced accordingly in the barrel 22 so that when the stopper 44 abuts the end wall 23 the proximal ends 70,72 of the locking element 60 are approximately at the location of the distal disc 48. The assembly of the syringe is then complete and the syringe is ready for use or storage. A needle cover 62 can be mounted to the distal end of the barrel onto the collar 32 to protect the needle cannula. The cover is removed prior to use.

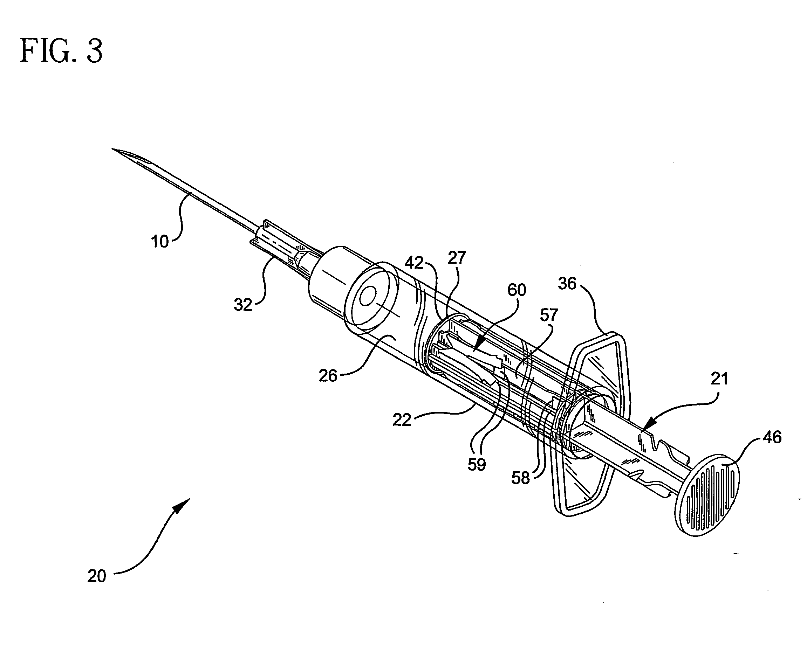

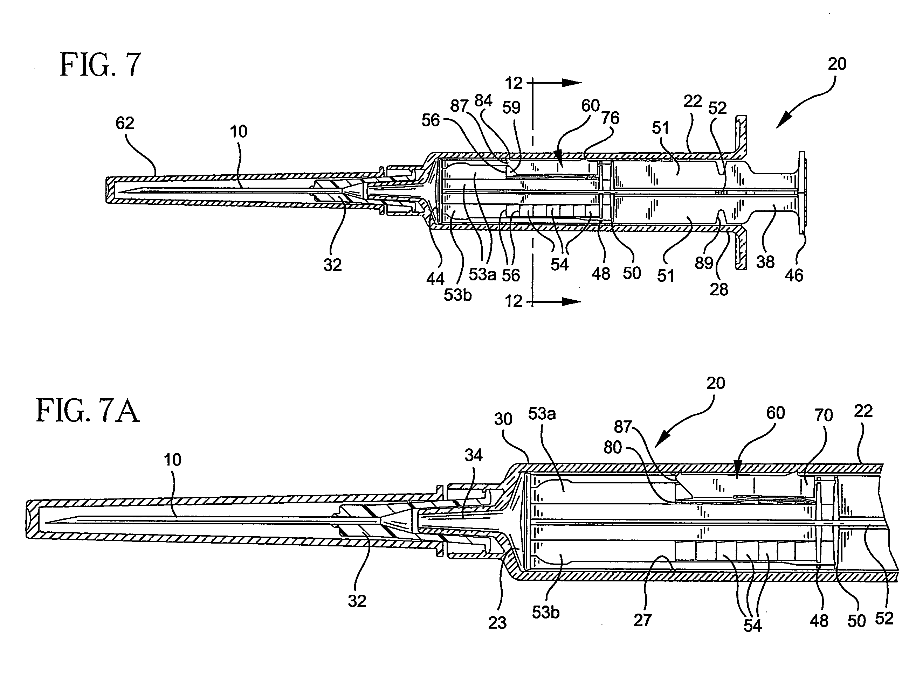

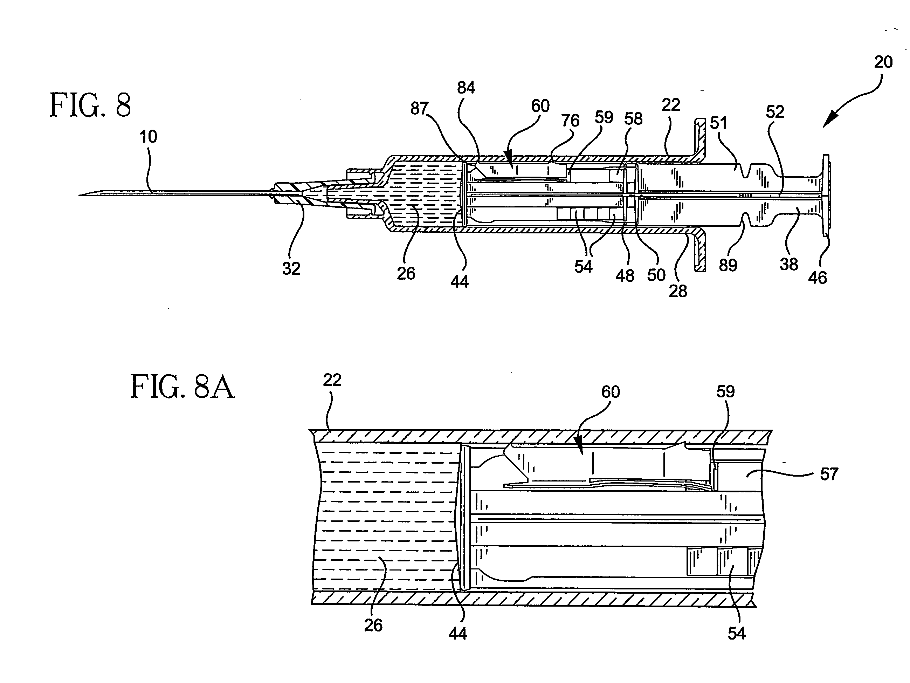

In use, plunger rod assembly 121 is retracted in the proximal direction from the position shown in Figs. 2, 7 and 7A to the position shown in Figs. 3, 8 and 8A in order to perform the first aspiration, drawing reconstitution fluid or diluent through needle cannula 10 and passageway 34 and into chamber 26 of barrel 22. The plunger is retracted proximally until the stopper 44 abuts the ring 27. Locking element 60 remains substantially stationary with respect to barrel 22 during such retraction, and the plunger rod 38 is moved proximally with respect to both barrel 22 and the locking element 60. This is due to the engagement of barbs 76, 78, 84, 86 with inside surface 24 of the barrel. The barbs are preferably made from a harder material than the barrel, which enhances their ability to resist proximal movement. As the plunger continues to move proximally, angled ends 70, 72 of legs 66, 68 of the locking element ride 60 over the proximal tooth 58 of the plunger rod during retraction thereof. The stopper 44 abuts the ring 27 before the locking element rides over the distal tooth 59 as shown in FIGS. 3, 8 and 8A. Thus, the locking element 60 does not pass over the distal tooth 59. The teeth 58, 59 are spaced apart to allow the locking element 60 to pass over only the proximal tooth 58 during the first aspiration while allowing the proper quantity of reconstitution fluid to be withdrawn into the syringe 20. For example, about 3.0-3.4ml of reconstitution fluid is required for a 3ml dose. Thus, the locking element 60 and the ring 27, which are appropriately spaced to provide the proper quantity of reconstitution fluid into the chamber 26, limit the retraction of the plunger rod 38 during aspiration.

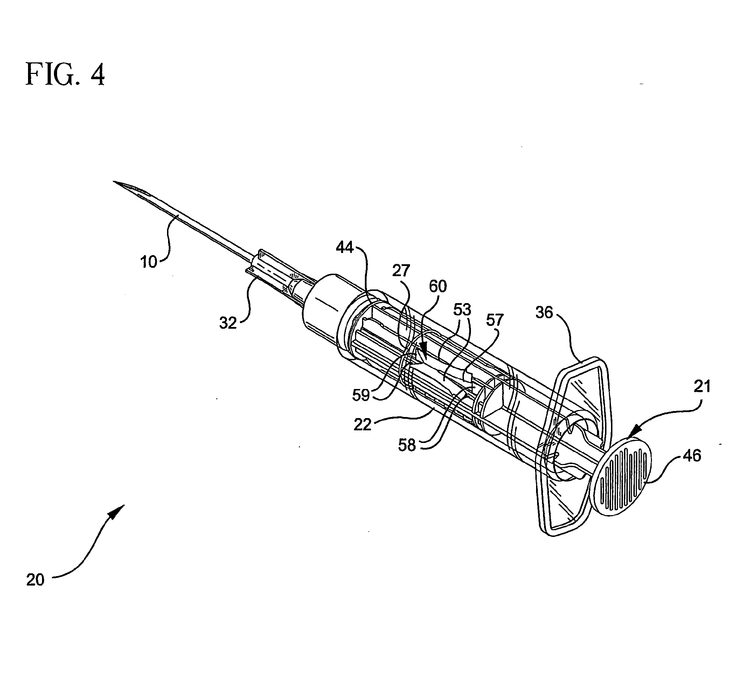

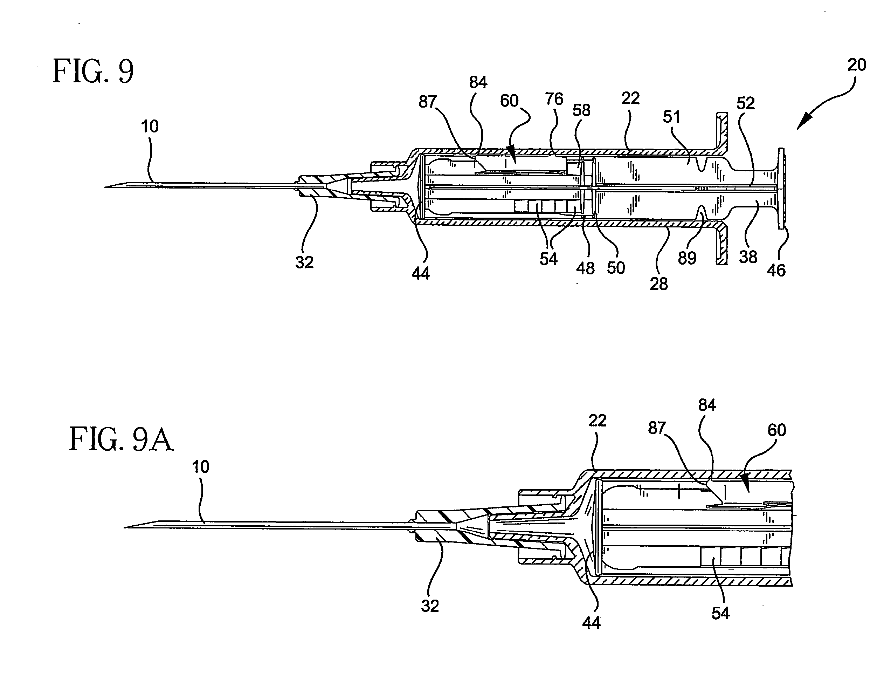

The reconstitution fluid may now be injected from chamber 26 into a lyophilized drug container (not shown). During the injection of the reconstitution fluid, the plunger is moved distally until the stopper 44 abuts end wall 23. This injection stroke empties the contents of the syringe into the drug container in order to reconstitute the drug. The locking element 60 remains substantially motionless, but may be slightly moved by the proximal tooth 58 during the injection of the reconstitution fluid into the lyophilized drug container as shown in FIGS. 4, 9 and 9A.

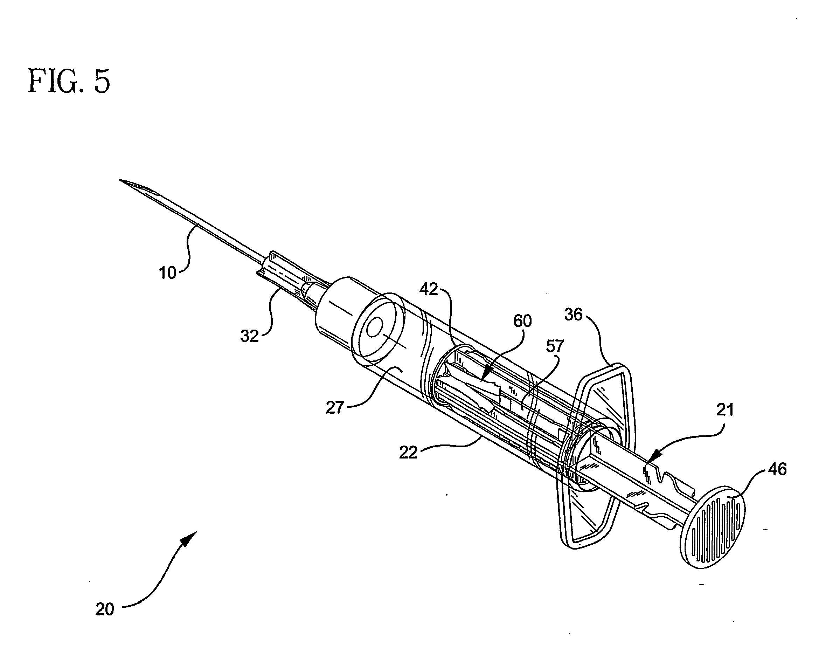

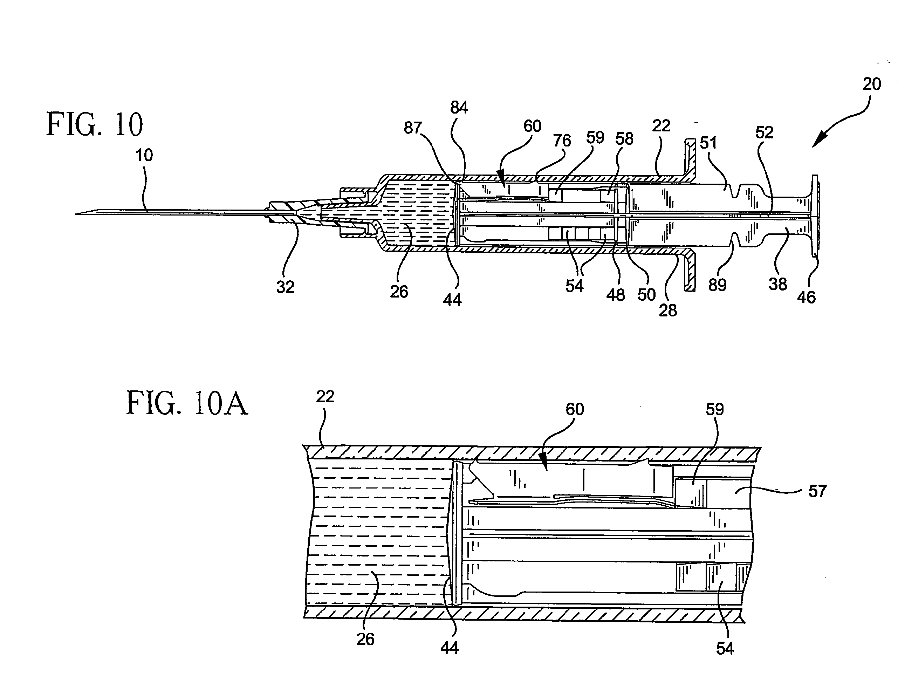

The reconstituted drug in the lyophilized drug container may now be drawn from the lyophilized drug container into the chamber 26 of the syringe. During the aspiration of the reconstituted drug, the locking element 60 passes over the proximal tooth 59 as shown in FIGS. 5, 10 and 10A. The plunger 38 is withdrawn proximally until the stopper 44 abuts the ring 27. The distal end of the locking element 60 now abuts the proximal side of the stopper 44. The user feels the engagement of both the locking element 60 and stopper 44 and the annular ring 27 and stopper 44. Cutting edges 87 do not penetrate the stopper 44 as a result of the forces exerted during normal use. As the locking element 60 cannot be moved proximally, further retraction of the plunger rod 38 is resisted. The amount of fluid that can be drawn into chamber 26 is accordingly limited by the distance between the proximal surface of the stopper 44 and the disc 48 as well as the length of the locking element 60 and the location of the ring 27. It will be appreciated that the distance between the stopper 44 and the relatively distal disc 48, the length of the locking element 60, the location of the ring 27 and the distance between the proximal and distal tooth 58, 59 can be chosen to meet the needs of particular applications. As discussed above, the locking element 60 is substantially immovable in the proximal direction within the barrel due to the engagement of the barbs with the inside surface of the barrel 22.

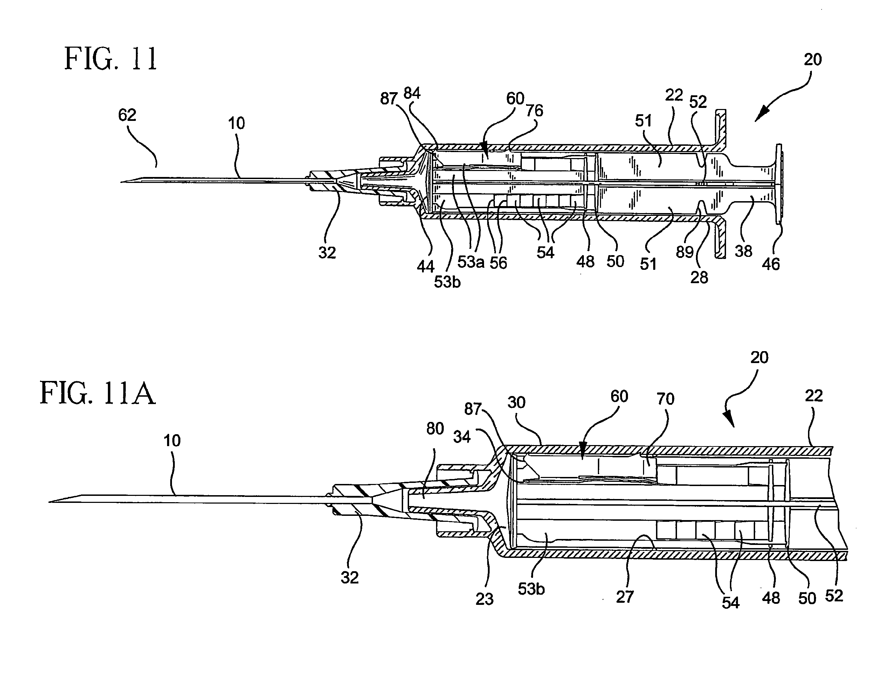

Once the reconstituted drug has been drawn into the barrel 22 from the lyophilized drug container, the needle cannula can be removed from the fluid source and used for injection. During the injection of a patient, plunger assembly 38 and locking element 60 both move distally from the positions shown in Figs. 10 and 10A to the positions shown in Figs. 11 and 11A. In Figs. 11 and 11A, stopper 44 again adjoins or engages the end wall 23 of barrel 22. At this point, locking element 60 remains positioned on the distal side of the distal tooth 54a and proximal end 70 of locking element 60 engages distal shoulder 56a of distal tooth 54a. Both plunger rod 38 and the locking element 60 are substantially immovable from their positions since barbs 76, 78, 84, 86 are engaged to inside surface 24, which resist proximal movement of plunger 38. Syringe assembly 20 accordingly cannot be reused. Should a person use extraordinary force in an attempt to retract the plunger rod assembly from the position shown in Figs. 11 and 11A, the cutting edges 87 at the distal end of the locking element 60 will penetrate the stopper, rendering it unusable. Disabling of the stopper 44 preferably occurs when the force exerted is approximately sufficient to dislodge the locking element 60 in the proximal direction, or a lesser force. As discussed above, simple engagement of the cutting edges 87 and stopper 44 should not compromise the integrity of the stopper 44.

An additional tamper-resistance feature is comprised of notches 89 in the plunger rod 38. If the plunger rod 38 is twisted forcefully, it will break prior to disablement of locking element 60.

If the locking element 60 is positioned within the recess 42 defined by the second pair of distal walls 53b and accordingly teeth 54, the syringe would only be usable for a single aspiration and injection stroke. The syringe would operate in the same way as fully disclosed in U.S. Patent Application Serial No. 10/254,924 filed September 25, 2002.

Thus, the same plunger assembly 38 can be used for single aspiration/injection procedures or procedures requiring two aspirations and injections. Alternatively, the plunger assembly 38 may be provided with distal walls 43 that each only include a proximal tooth 58 and a distal tooth 59. In such a device, the locking element 60 may be placed in any recess 42 in order to perform a reconstitution procedure.

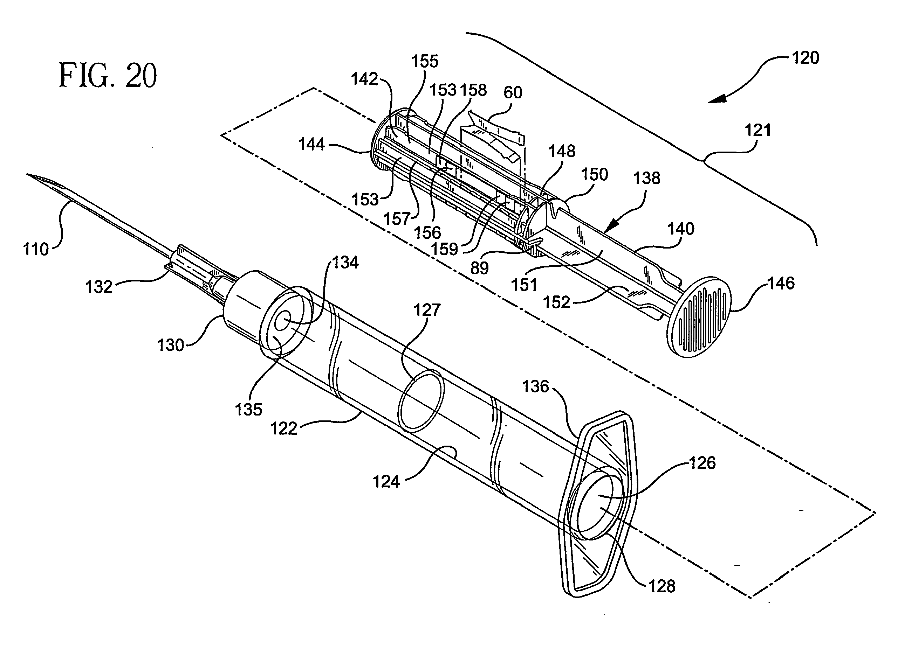

Referring now to FIGS. 20-25, another embodiment of the invention is illustrated. This embodiment of the invention automatically disables the syringe after a two stroke procedure is performed, for applications in which the volume of the reconstitution fluid or diluent necessary to reconstitute the drug exceeds the volume of the actual dose to be delivered. For example, 3.2ml of reconstitution fluid can be used to reconstitute a drug and then the practitioner may choose to deliver 2, 3, or 4 ml of drug. The treatment of tuberculosis, for example, require the reconstitution of 1 ml of Streptomycin with 3.2ml of diluent, to form 4 ml of drug at a concentration of 250 mg/ml. The standard doses are then determined by body mass of the patient, typically 2 ml, 3 ml, or 4 ml of drug.

FIG. 20 shows a single use syringe assembly 120 including a barrel 122 having an inside surface 124 defining a chamber 126 for retaining fluid. The inside surface 124 of the barrel further defines an annular ring 127 projecting therefrom. The barrel 122 includes an open proximal end 128 and a distal end 130 having a collar 132defining a passageway 134 therethrough in communication with the chamber. A needle cannula 110 projects outwardly from the distal barrel end and is in fluid communication with the chamber 126. The needle cannula 110 and the collar 132 as show in the FIGS. are integral with the barrel. It will be appreciated that the invention could be applied to syringe assemblies having permanently removable needles or needle/hub assemblies, or fixed or removable blunt cannulas, or standard luer locking assemblies. While intended for reconstitution of vaccines, the syringe assembly can be used for general curative purposes or other purposes.

The proximal end 128 of the barrel 122 includes a flange 136 to facilitate handling and positioning of the syringe assembly 120 and to maintain the relative position of the barrel 122 with respect to plunger rod 138 during filling and medication administration.

The plunger rod 138 used in the syringe assembly 120 includes an elongate body portion 140 including a plurality of elongate recesses 142 running substantially parallel to the longitudinal axis of rotation thereof. The distal end of the elongate body portion 140 includes an integral stopper 144. A disc-shaped flange 146 is provided at the proximal end of the plunger rod 138 for allowing the user to apply the force necessary to move the plunger rod 138 with respect to the barrel 122.

The elongate body portion 140 includes a pair of discs 148, 150 intermediate to the proximal and distal ends thereof. The sections between the relatively proximal disc 150 and the flange 146 and the relatively distal disc 148 and the stopper 144 include radially extending walls 151, 152, both of which preferably traverse the longitudinal axis of rotation of the plunger rod 138. Additional pairs of distal walls 153 resembling fins extend perpendicularly from both sides of one wall 152 of the radially extending walls in the section between the distal disc 148 and stopper 144. In this embodiment, as shown in the FIGS., there are four pairs of distal walls 153 including wall faces 155, 157. One pair of distal walls 153 extend perpendicularly from the upper side of wall 152 and another pair extend from the lower side of wall 152. The third and fourth pairs are mirror images of the first and second extending from the wall 152 on the side opposite from the first and second pairs beyond wall 151.

In a preferred embodiment, each of the distal walls 153 is substantially parallel to each other. The areas between each of the pairs of walls could be filled in to provide additional rigidity if necessary. In contrast to the previous embodiment, the embodiment of the invention disclosed in FIGS. 20-25 include a plurality of distal teeth 158 and a plurality of proximal teeth 159 rather than just a single proximal tooth 58 and a single distal tooth 59. As shown in FIGS. 20-25, there are three proximal teeth 158 and three distal teeth 159, but the number of proximal teeth and distal teeth may be modified according to the dosage of medication necessary. The distal teeth 158 and proximal teeth 159 are formed on selected surfaces of the walls 153. Each of the distal and proximal teeth 158, 159 includes a corresponding distally facing surface or shoulder 156. The wall surfaces 155, 157 including the teeth, converge along an imaginary line that runs substantially parallel to the longitudinal axis of the plunger rod, but radially displaced therefrom. The wall surfaces 155, 157 define recesses 142 for positioning of a locking element 60. While four recesses are provided, a greater or lesser number may be employed. It will be appreciated that the recesses 142 can be formed by surfaces that actually meet along the line of convergence. It will further be appreciated that while the plunger rod as shown and described herein is of integral construction, it may in fact be comprised of two or more separate elements. The stopper 144 may, for example, be a separate component made from a material that is different from the material comprising the remainder of the plunger rod. Preferably, the syringe barrel is comprised of polypropylene, and contains an internal lubricant and the plunger rod is comprised of polyethylene.

According to this embodiment of the invention, the dimensions of the syringe barrel are that of a standard 10 ml 2 piece syringe. In a preferred embodiment, the length of the plunger rod from the distal disc 148 to the stopper is about 55.7 mm; the total-length-of the proximal teeth is about 16.6 mm and length of area 162 is about 8.0 mm. The annular ring 127 is located on the inside of the barrel 122 at a position approximately 33.1mm from the end wall 135 at the distal end130 of the barrel. These measurements are merely illustrative measurements of the preferred embodiment to allow for delivery of a drug typical in the tuberculosis regimen. For other applications requiring a two-stroke procedure, the location and lengths of the proximal teeth and distal teeth, as well as the location of the annular ring 127 can be adjusted accordingly.

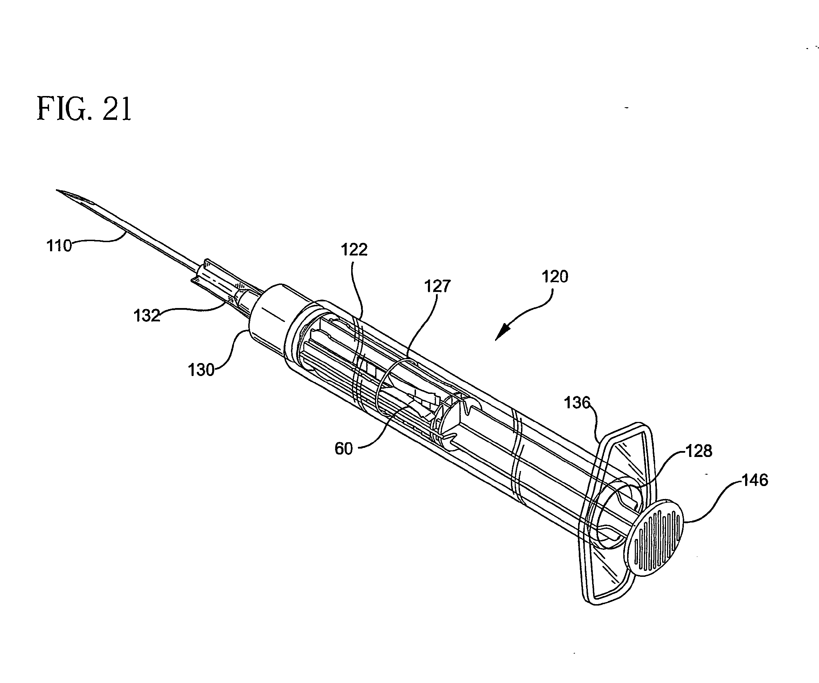

The syringe assembly 120 is easily constructed from the component parts thereof and packaged as shown in FIGS. 20 and 21. First, the locking element 60 is positioned in one of recesses 142 in the plunger rod 138 such that the angled end portions of the legs 66, 68 adjoin the relatively distal disk 48. The legs 66, 68 and spring member extend proximally, and the barbs 76, 78, 84, 86 are all angled proximally with respect to the plunger rod 38. The plunger rod/locking element assembly 121 is then inserted into the barrel 122 through the proximal end thereof.

As the assembly 121 is moved distally within the barrel, the angular orientation of the barbs allows them to slide along while engaging inside surface 124 of the barrel. The locking element 60 moves distally with the plunger rod 138 due to the engagement of the ends of legs 66, 68 with the disc 148. The gap 74 is maintained between the legs 66, 68 even after installation of the locking element. The maintenance of the gap and the relatively long lengths of the legs 66,68, which act as cantilever springs, provide a relatively reduced force on the barrel and facilitate use and installation. The plunger rod/locking element assembly 121 is moved distally until the stopper 144 engages the end wall 135 of the barrel 122 and the distal portion of the locking element 60, including the cutting edge 87 and barbs 84 abut the annular ring 127. The ring 127 is spaced accordingly in the barrel 122 so that when the stopper 44 abuts the end wall 135 the proximal ends 70, 72 of the locking element 60 are approximately at the location of the distal disc 148. The assembly of the syringe is then complete and the syringe is ready for use or storage. A needle cover can be mounted to the distal end of the barrel onto the collar 132 to protect the needle cannula. The cover is removed prior to use.

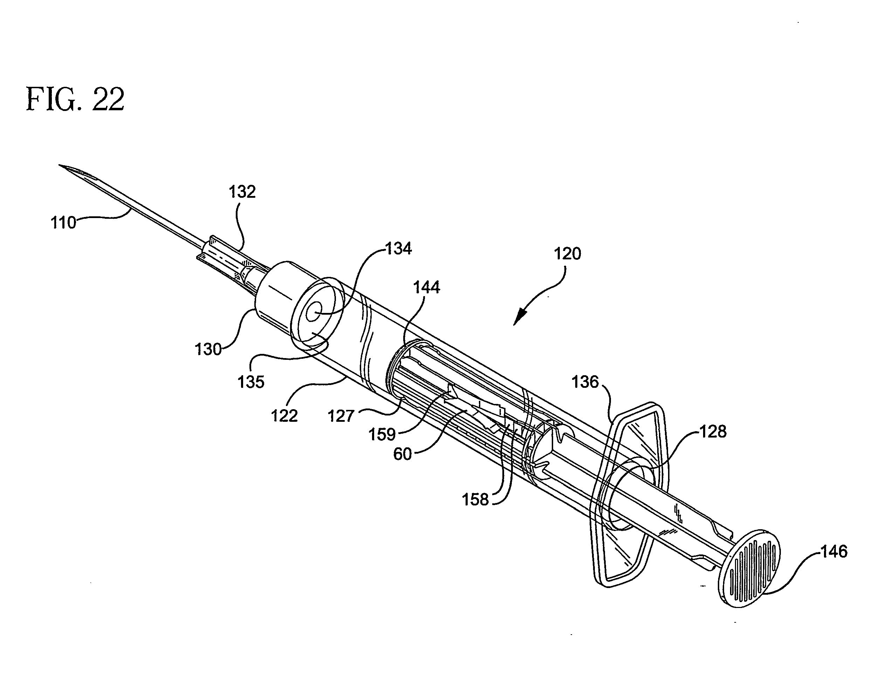

In use, plunger rod assembly 121 is retracted in the proximal direction from the position shown in FIG. 21 to the position shown in FIG. 22 in order to perform the first aspiration, drawing reconstitution fluid or diluent through needle cannula 110 and passageway 134 and into chamber 126 of barrel 122. The plunger is retracted proximally until the stopper 144 abuts the ring 127. Locking element 60 remains substantially stationary with respect to barrel 122 during such retraction, and the plunger rod 138 is moved proximally with respect to both barrel 122 and the locking element 60. This is due to the engagement of barbs 76, 78, 84, 86 with inside surface 124 of the barrel. The barbs are preferably made from a harder material than the barrel, which enhances their ability to resist proximal movement. As the plunger continues to move proximally, angled ends 70, 72 of legs 66, 68 of the locking element ride 60 over all of the plurality of proximal tooth 58 of the plunger rod during retraction thereof. The stopper 144 abuts the ring 127 before the locking element rides the plurality of distal teeth 159 as shown in FIG. 22. Thus, the locking element 60 does not pass over any of the distal teeth 159. The proximal teeth 158 and the distal teeth 159 are spaced apart to allow the locking element 60 to pass over only the proximal teeth 158 during the first aspiration while allowing the proper quantity of reconstitution fluid to be withdrawn into the syringe 120. Thus, the locking element 60 and the ring 127, which are appropriately spaced to provide the proper quantity of reconstitution fluid into the chamber 126, limit the retraction of the plunger rod 138 during aspiration.

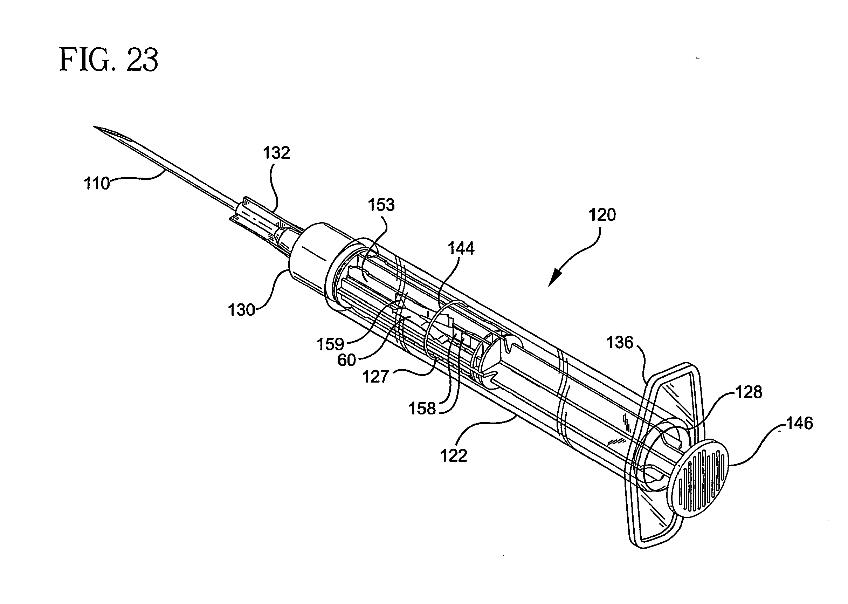

The reconstitution fluid may now be injected from chamber 126 into a lyophilized drug container (not shown). During the injection of the reconstitution fluid, the plunger is moved distally until the stopper 144 abuts end wall 135, as shown in FIG. 23. This injection stroke empties the contents of the syringe into the drug container in order to reconstitute the drug. The locking element 60 travels with the plunger 138 due to the primal end of the locking element contacting the distal most proximal tooth 158, as shown in FIG. 23.

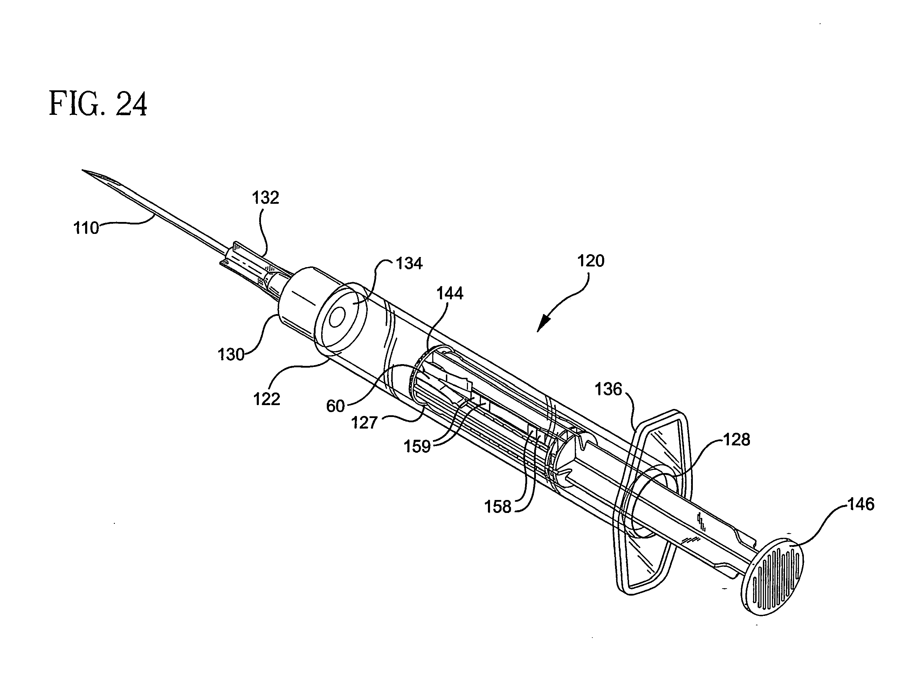

The reconstituted drug in the lyophilized drug container may now be drawn from the lyophilized drug container into the chamber 126 of the syringe. During the aspiration of the reconstituted drug, the locking element 60 passes over the proximal teeth 159 as shown in FIG. 24. The plunger 138 is withdrawn proximally until the stopper 144 abuts the ring 127. The distal end of the locking element 60 now abuts the proximal side of the stopper 144. The user feels the engagement of both the locking element 60 and stopper 144 and the annular ring 127 and the stopper 144. Cutting edges 87 do not penetrate the stopper 144 as a result of the forces exerted during normal use. As the locking element 60 cannot be moved proximally, further retraction of the plunger rod 138 is resisted. The amount of fluid that can be drawn into chamber 126 is accordingly limited by the distance between the proximal surface of the stopper 144 and the disc 148 as well as the length of the locking element 160 and the location of the ring 127. It will be appreciated that the distance between the stopper 144 and the distal disc 148, the length of the locking element 60, the location of the ring 127, the length of the proximal and distal teeth 158, 159 and the distance between the proximal and distal teeth 158, 159 can be chosen to meet the needs of particular applications.

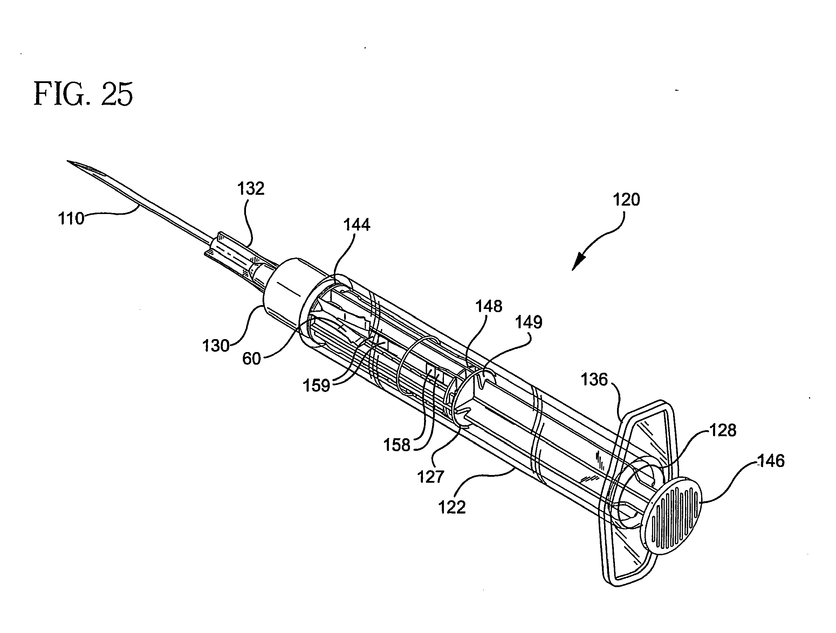

Once the reconstituted drug has been drawn into the barrel 122 from the lyophilized drug container, the needle cannula can be removed from the fluid source and used for injection. During the injection of a patient, the plunger 138 and the locking element 60 both move distally from the position shown in FIG. 24 to the position shown in FIG. 25. As shown in FIG. 25, the stopper 44 again adjoins or engages the end wall 135 of barrel 122. At this point, locking element 60 remains positioned on the distal side of the distal teeth 159 and proximal end 70 of locking element 60 engages distal shoulder 56 of distal teeth 159. Both plunger rod 138 and the locking element 60 are substantially immovable from their positions since barbs 76, 78, 84, 86 are engaged to inside surface 124, which resist proximal movement of plunger 38. Syringe assembly 120 accordingly cannot be reused. Should a person use extraordinary force in an attempt to retract the plunger rod assembly from the position shown in FIG. 25, the cutting edges 87 at the distal end of the locking element 60 will penetrate the stopper, rendering it unusable. Disabling of the stopper 144 preferably occurs when the force exerted is approximately sufficient to dislodge the locking element 60 in the proximal direction, or a lesser force. As discussed above, simple engagement of the cutting edges 87 and stopper 144 should not compromise the integrity of the stopper 144.

The syringe barrel of the present invention may be constructed of a wide variety of rigid materials with thermoplastic materials such as polypropylene and polyethylene being preferred. Similarly, thermoplastic materials such as polypropylene, polyethylene and polystyrene are preferred for the plunger rod and integral stopper. A wide variety of materials such as natural rubber, synthetic rubber, thermoplastic elastomers and combinations thereof are suitable for the stopper if the stopper is manufactured as a separate component. The choice of stopper material will depend on compatibility with the medication being used.

As previously recited, it is preferable that the locking element 60 be fabricated from a material, which is harder than the barrel 22 so that the locking barbs may effectively engage the barrel 22. Resilient spring like properties are also desirable along with low cost, dimensionally consistent fabrication. With this in mind, sheet metal is the preferred material for the locking element 60 with stainless steel being preferred for medical applications. Although the locking element of the preferred embodiment is fabricated from a single sheet, it is within the purview of the instant invention to include locking elements made of other forms and/or containing multiple parts. Locking elements having structures other than that shown and described herein could also be successfully employed. One such locking element is disclosed in U.S. Patent No. 5,989,219. The distal end of the locking element disclosed in the patent could be provided with a cutting edge similar to those described above. Alternatively, barbs (not shown) could be provided at the distal end of the locking element for rendering the stopper unusable.

The syringe barrel employed in accordance with the invention may have a varying wall thickness along its length. The portion of the barrel used for containing medication could be relatively thin and resilient to ensure proper sealing with the stopper. The remainder of the barrel could be relatively thick and less resilient such that it would tend to crack if squeezed by pliers or another device used for attempted tampering. Sufficient barrel crystallinity is desirable in the area of the locking element to cause this area to crack upon deformation of the syringe barrel to an extent that would permit retraction of the plunger rod with the locking element.

Thus, it can be seen that the present invention provides a simple, reliable, easily fabricated, single use syringe which becomes inoperable or incapable of further use without any additional act on the part of the user. It further allows the use of a locking element of the same size that is used on smaller or larger syringes. It even further allows use of a plunger with a variety recesses, which contain teeth arrangements to achieve the desired dosage range and plunger retraction sequences.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the invention, as defined by the appended claims.

## Claims

1. A syringe assembly comprising:
a syringe barrel having an inside surface defining a chamber, an open end, and a distal end;
a plunger rod extending within said syringe barrel, said plunger rod including an elongate body portion defining a longitudinal axis, a stopper at a distal end of said elongate body portion, a disc, and at least one axially extending recess formed by a pair of surfaces radially displaced from the longitudinal axis of said elongate body portion, each of said surfaces defining at least one proximal tooth and at least one distal tooth, said plunger rod being substantially immovable after performing a first retraction stroke in the direction of said open end, a first injection stroke in the direction of said distal end, a second retraction stroke in the direction of said open end and a second injection stroke in the direction of said distal end; and
a locking element slidably positioned within said recess, said locking element engaging said inside surface of said syringe barrel such that said locking element is substantially immovable in the direction of the open end of said syringe barrel, said locking element further being engageable with said at least one proximal tooth during said first injection stroke of said plunger rod and said locking element being engageable with said at least one distal tooth during said second injection stroke of said plunger rod.

2. The syringe assembly of claim 1, wherein said barrel further defines an annular ring for limiting movement of said plunger rod in the direction of said open end.

3. The syringe assembly of claim 2, wherein said plunger rod movement is limited in the direction of the open end during said first retraction stroke when said stopper abuts a said annular ring.

4. The syringe assembly of claim 2, wherein said plunger rod movement is limited in the direction of the open end during said second retraction stroke when said stopper abuts a said annular ring.

5. The syringe assembly of claim 1, wherein said at least one proximal tooth comprises a plurality of proximal teeth and said at least one distal tooth comprises a plurality of distal teeth.

6. The syringe assembly of claim 1, wherein said at least one proximal tooth comprises three teeth.

7. The syringe assembly of claim 1 wherein said locking element is comprised of an integral, resilient metal structure, said locking element being positioned such that said plunger rod can be moved proximally with respect to said locking element.

8. The syringe assembly of claim 1 wherein said locking element includes one or more proximally extending barbs engaging said inside surface of said syringe barrel, and said locking element and stopper are positioned such that said plunger rod can be moved proximally with respect to said locking element.

9. The syringe assembly of claim 8 wherein said locking element includes a body portion having a distal end and proximal end, said body portion of said locking element being generally V-shaped and engageable with each of said pair of surfaces.

10. The syringe assembly of claim 1 wherein said elongate body portion of said plunger rod includes a plurality of radially extending walls that converge near said longitudinal axis and a first additional wall extending from one of said radially extending walls in a non-radial direction, said first additional wall defining one of said surfaces forming said recess.

11. The syringe assembly of claim 10 including a second additional wall extending from one of said radially extending walls, said second additional wall defining one of said surfaces forming said recess.

12. The syringe assembly of claim 1 wherein said elongate body portion of said plunger rod includes a first wall proximal to said stopper and a plurality of second walls projecting from a first side of first wall, two of said second walls defining said surfaces forming said recess.

13. The syringe assembly of claim 1 wherein said annular ring is spaced a distance from said distal end such that said distance defines a volume of said chamber to be substantially equal to a desired reconstitution dose.

14. The syringe assembly of claim 1 further comprising a needle cannula attached to said distal end of said barrel and in fluid communication with said barrel.

## Patentansprüche

1. Spritzenanordnung mit:
einem Spritzenzylinder mit einer Innenfläche, die eine Kammer begrenzt, einem offenen Ende und einem distalen Ende;
einer Kolbenstange, die sich in dem Spritzenzylinder erstreckt, wobei die Kolbenstange einen langgestreckten Körperteil mit einer Längsachse, einen Stopfen an einem distalen Ende des langgestreckten Körpers, eine Scheibe und mindestens eine axial verlaufende Ausnehmung aufweist, welche durch zwei radial gegenüber der Längsachse des langgestreckten Körperteils versetzte Flächen gebildet ist, wobei jede der Flächen mindestens einen proximalen Zahn und mindestens einen distalen Zahn bildet, wobei die Kolbenstange nach dem Durchführen eines ersten Rückzugshubs in Richtung des offenen Endes, eines ersten Injektionshubs in Richtung des distalen Endes, eines zweiten Rückzugshubs in Richtung des offenen Endes und eines zweiten Injektionshubs in Richtung des distalen Endes im wesentlichen unbewegbar ist; und
einem Verriegelungselement, das gleitend verschiebbar in der Ausnehmung angeordnet ist, wobei das Verriegelungselement an der Innenfläche des Spritzenzylinders derart angreift, dass das Verriegelungselement in Richtung des offenen Endes des Spritzenzylinders im wesentlichen unbewegbar ist, wobei das Verriegelungselement ferner während des ersten Injektionshubs der Kolbenstange in Eingriff mit dem mindestens einen proximalen Zahn bringbar ist, und das Verriegelungselement während des zweiten Injektionshubs der Kolbenstange mit dem wenigstens einen distalen Zahn in Eingriff bringbar ist.

2. Spritzenanordnung nach Anspruch 1, bei welcher der Zylinder einen kranzförmigen Ring zum Begrenzen der Bewegung der Kolbenstange in Richtung des offenen Endes aufweist.

3. Spritzenanordnung nach Anspruch 2, bei der die Bewegung der Kolbenstange in Richtung des offenen Endes während des ersten Rückzughubs durch das Anstoßen des Stopfens an den kranzförmigen Ring begrenzt ist.

4. Spritzenanordnung nach Anspruch 2, bei der die Bewegung der Kolbenstange in Richtung des offenen Endes während des zweiten Rückzugshubs durch das Anstoßen des Stopfens an den kranzförmigen Ring begrenzt ist.

5. Spritzenanordnung nach Anspruch 1, bei welcher der mindestens eine proximale Zahn mehrere proximale Zähne umfasst und der mindestens eine distale Zahn mehrere distale Zähne umfasst.

6. Spritzenanordnung nach Anspruch 1, bei welcher der mindestens eine proximale Zahn drei Zähne umfasst.

7. Spritzenanordnung nach Anspruch 1, bei der das Verriegelungselement aus einer einstückigen, elastischen Metallstruktur besteht, wobei das Verriegelungselement derart angeordnet ist, dass die Kolbenstange in bezug auf das Verriegelungselement proximal bewegbar ist.

8. Spritzenanordnung nach Anspruch 1, bei der das Verriegelungselement einen oder mehrere sich proximal erstreckende Haken aufweist, die an der Innenfläche des Spritzenzylinders angreifen, und das Verriegelungselement und der Stopfen derart positioniert sind, dass die Kolbenstange in bezug auf das Verriegelungselement proximal bewegbar ist.

9. Spritzenanordnung nach Anspruch 8, bei der das Verriegelungselement einen Körperteil mit einem distalen Ende und einem proximalen Ende aufweist, wobei der Körperteil des Verriegelungselements im wesentlichen V-förmig ist und in Angriff an jeder der genannten beiden Flächen bringbar ist.

10. Spritzenanordnung nach Anspruch 1, bei welcher der langgestreckte Körperteil der Kolbenstange mehrere sich radial erstreckende Wände, die nahe der Längsachse konvergieren, und eine erste zusätzliche Wand aufweist, die sich von einer der sich radial erstreckenden Wände in nicht-radialer Richtung erstreckt, wobei die erste zusätzliche Wand eine der die Ausnehmung bildenden Flächen bildet.

11. Spritzenanordnung nach Anspruch 10, mit einer zweiten zusätzlichen Wand, die sich von einer der sich radial erstreckenden Wände aus erstreckt, wobei die zweite zusätzliche Wand eine der die Ausnehmung bildenden Flächen bildet.

12. Spritzenanordnung nach Anspruch 1, bei welcher der langgestreckte Körperteil der Kolbenstange eine erste Wand nahe dem Stopfen und mehrere zweite Wände aufweist, die von einer ersten Seite der ersten Wand abstehen, wobei zwei der zweiten Wände die die Ausnehmung bildenden Flächen bilden.

13. Spritzenanordnung nach Anspruch 1, bei welcher der kranzförmige Ring von dem distalen Ende derart beabstandet ist, dass der Abstand ein Volumen der Kammer bildet, das im wesentlichen gleich einer gewünschten Rekonstitutionsdosis ist.

14. Spritzenanordnung nach Anspruch 1, ferner mit einer an dem distalen Ende des Zylinders und in Fluidverbindung mit dem Zylinder angebrachten Nadelkanüle.

## Revendications

1. Ensemble de seringue comprenant :
un corps cylindrique de seringue ayant une surface interne définissant une chambre, une extrémité ouverte et une extrémité distale ;
une tige de piston s'étendant à l'intérieur dudit corps cylindrique de seringue, ladite tige de piston comprenant une partie de corps allongé définissant un axe longitudinal, une butée au niveau d'une extrémité distale de ladite partie de corps allongé, un disque, et au moins un évidement s'étendant de manière axiale formé par une paire de surfaces déplacées radialement par rapport à l'axe longitudinal de ladite partie de corps allongé, chacune desdites surfaces définissant au moins une dent proximale et au moins une dent distale, ladite tige de piston étant sensiblement immobile après la réalisation d'une première course de rétraction dans la direction de ladite extrémité ouverte, d'une première course d'injection dans la direction de ladite extrémité distale, d'une seconde course de rétraction dans la direction de ladite extrémité ouverte et d'une seconde course d'injection dans la direction de ladite extrémité distale ; et
un élément de blocage positionné de manière coulissante à l'intérieur dudit évidement, ledit élément de blocage se mettant en prise avec ladite surface interne dudit corps cylindrique de seringue de sorte que ledit élément de blocage est sensiblement immobile dans la direction de l'extrémité ouverte dudit corps cylindrique de seringue, ledit élément de blocage pouvant en outre se mettre en prise avec ladite au moins une dent proximale pendant ladite première course d'injection de ladite tige de piston , et ledit élément de blocage pouvant se mettre en prise avec ladite au moins une dent distale pendant ladite seconde course d'injection de ladite tige de piston.

2. Ensemble de seringue selon la revendication 1, dans lequel ledit corps cylindrique définit en outre une bague annulaire pour limiter le mouvement de ladite tige de piston dans la direction de ladite extrémité ouverte.

3. Ensemble de seringue selon la revendication 2, dans lequel ledit mouvement de la tige de piston est limité dans la direction de l'extrémité ouverte pendant ladite première course de rétraction lorsque ladite butée vient en butée contre ladite bague annulaire.

4. Ensemble de seringue selon la revendication 2, dans lequel ledit mouvement de la tige de piston est limité dans la direction de l'extrémité ouverte pendant ladite seconde course de rétraction lorsque ladite butée vient en butée contre ladite bague annulaire.

5. Ensemble de seringue selon la revendication 1, dans lequel ladite au moins une dent proximale comprend une pluralité de dents proximales et ladite au moins une dent distale comprend une pluralité de dents distales.

6. Ensemble de seringue selon la revendication 1, dans lequel ladite au moins une dent proximale comprend trois dents.

7. Ensemble de seringue selon la revendication 1, dans lequel ledit élément de blocage est composé d'une structure métallique élastique solidaire, ledit élément de blocage étant positionné de sorte que ladite tige de piston peut être déplacée de manière proximale par rapport audit élément de blocage.

8. Ensemble de seringue selon la revendication 1, dans lequel ledit élément de blocage comprend un ou plusieurs ardillons s'étendant de manière proximale se mettant en prise avec ladite surface interne dudit corps cylindrique de seringue, et ledit élément de blocage et ladite butée sont positionnés de sorte que ladite tige de piston peut être déplacée de manière proximale par rapport audit élément de blocage.

9. Ensemble de seringue selon la revendication 8, dans lequel ledit élément de blocage comprend une partie de corps ayant une extrémité distale et une extrémité proximale, ladite partie de corps dudit élément de blocage ayant généralement une forme en V, et pouvant se mettre en prise avec chacune de ladite paire de surfaces.

10. Ensemble de seringue selon la revendication 1, dans lequel ladite partie de corps allongée de ladite tige de piston comprend une pluralité de parois s'étendant de manière radiale qui convergent à proximité dudit axe longitudinal, et une première paroi supplémentaire s'étendant à partir de l'une desdites parois s'étendant de manière radiale dans une direction non radiale, ladite première paroi supplémentaire définissant l'une desdites surfaces formant ledit évidement.

11. Ensemble de seringue selon la revendication 10, comprenant une seconde paroi supplémentaire s'étendant à partir de l'une desdites parois s'étendant de manière radiale, ladite seconde paroi supplémentaire définissant l'une desdites surfaces formant ledit évidement.

12. Ensemble de seringue selon la revendication 1, dans lequel ladite partie de corps allongé de ladite tige de piston comprend une première paroi proximale par rapport à ladite butée et une pluralité de secondes parois faisant saillie à partir d'un premier côté de la première paroi, deux desdites secondes parois définissant lesdites surfaces formant ledit évidement.

13. Ensemble de seringue selon la revendication 1, dans lequel ladite bague annulaire est à une certaine distance de ladite extrémité distale de sorte que ladite distance définit un volume de ladite chambre pour être sensiblement identique à une dose de reconstitution souhaitée.

14. Ensemble de seringue selon la revendication 1, comprenant en outre une canule à aiguille fixée sur ladite extrémité distale dudit corps cylindrique et en communication de fluide avec ledit corps cylindrique.
